# EUROPEAN PATENT APPLICATION

(11) **EP 4 516 889 A1**
(43) Date of publication of application: **05.03.2025**
(21) Application number: 23193832.5
(22) Date of filing: 29.08.2023
(51) Int. Cl.: C12M 1/00, A23L 3/32, F04D 29/02, F04D 29/42

(54) **PREVENTION OF MICROBIOLOGICAL GROWTH IN PROCESSING SYSTEMS**

(71) Applicant: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(57) **Abstract**

A system (10) is proposed that comprises: a fluid-processing equipment (12), a pipe arrangement (14), and an electric power source (16). The fluid-processing equipment (12) has a housing (18) arranged to hold a fluid, and the pipe arrangement (14) is arranged to lead the fluid to and from the housing (18). The housing (18) is electrically conductive, the pipe arrangement (14) is electrically conductive and electrically coupled to the housing (18), and the power source (16) is arranged to supply an electric current. The housing (18), pipe arrangement (14), and the power source (16) are operationally coupled in an electric circuit (20) connected to ground and arranged to lead the current to or from ground via the pipe arrangement (14) and the housing (18), and the current is configured to inhibit microbiological growth within the housing (18).

## Description

### Technical field

The technology proposed herein relates to the prevention of microbiological growth in fluid-processing systems, such as food-processing systems.

### Background

Microbiological growth in food processing equipment is a recognized problem, for example in diary processing. The feed that is processed has a high nutrient content and the rate of microbiological growth in the equipment is typically high. Extensive biofilms can form in a few hours. Frequent cleaning is typically required to prevent a drop in quality of the produced product or in the performance of the equipment. For example, microbiological growth may contaminate the produced product, or it may obstruct the flow of the fluid within the equipment. Typically, production must be interrupted for cleaning and chemicals are commonly used, which contributes to a decreased efficiency and increased production costs.

Microbiological growth is particularly a problem in equipment that that cannot easily be disassembled for manual cleaning. Equipment having moving components, such as centrifuge pumps and centrifuge separators are typically difficult to disassemble.

### Object of the proposed technology

The proposed technology aims at preventing or reducing microbiological growth, such as the formation of biofilms, in systems with fluid-processing equipment. A particular object of the proposed technology is to provide a system for reducing microbiological growth that is easy to install.

### Summary

In a first aspect of the proposed technology, a system is proposed that comprises: a fluid-processing equipment, a pipe arrangement, and an electric power source, or electric power supply. The fluid-processing equipment has, or forms, a housing arranged to hold, or contain, a fluid, the pipe arrangement is arranged to lead the fluid to and from the housing. The housing is electrically conductive, the pipe arrangement is electrically conductive and electrically coupled to the housing, and the power source is arranged to supply an electric current. The housing, pipe arrangement, and the power source are operationally coupled in an electric circuit connected to ground and arranged to lead the current to or from ground via the pipe arrangement and the housing. The current is configured to inhibit, reduce, or prevent, microbiological growth within the housing, or on, the housing.

It is understood that the intention of the system is to prevent microbiological growth in the housing. It is further understood that the fluid-processing equipment may be, or form part of, a food-processing equipment. It is further understood that the housing is in fluid communication with the pipe arrangement. It is understood that microbiological growth may be inhibited also within the pipe arrangement, since it forms part of the electric circuit. The proposed system has the advantage of an easy installation.

It is understood that the fluid-processing equipment may be arranged to change inherent properties of the fluid. For example, it may be or comprise a filter arranged to filter the fluid, a separator arranged to form a lighter phase and a heavier phase of the fluid, or a homogenizer arranged to change the size of particles in the fluid.

It is understood that the fluid-processing equipment may be arranged to change static or dynamic properties of the fluid. For example, it may be or comprise a pump arranged to increase a pressure of the fluid, a pump arranged to increase a flow rate of the fluid, a valve arranged to regulate a flow of the fluid, or a heater or cooler arranged to change the temperature of the fluid.

It is understood that the housing is arranged to allow a flow of the fluid through the housing. Worded differently, the fluid-processing equipment and the pipe arrangement may be arranged to allow a flow of the fluid through the housing.

It is further understood that the housing has a portion that is exposed to the fluid, and the electric circuit may be arranged to lead the current, or a part of the current, supplied by the electric power source through, or via, the portion exposed to the fluid. It is further understood that the current is adapted to inhibit microbiological growth on the housing or on the portion of the housing exposed to the fluid.

It is specified that the electric circuit is arranged to lead the current to or from ground via the pipe arrangement and the housing. Effectively, this means that ground forms part of the electric circuit. The electric circuit may be connected to ground. Worded differently, the pipe arrangement and the housing may be electrically coupled to the power source via ground, or the electric circuit may incorporate ground. It is understood that the ground specified above is electric ground. Ground may be a signal ground or earth ground. For example, ground may incorporate an electrically conductive structure connected to and supporting the housing.

It is understood that the housing extends in all dimensions and that the current may be evenly or unevenly distributed in the housing. It is further understood that at least a portion of the pipe arrangement and a portion of the housing forms part of the electric circuit. This means that the current passes through, or via, these portions. The complete pipe arrangement and the complete housing may form part of the circuit, which means that the current passes through the complete pipe arrangement and the complete housing.

Throughout these specifications, if a first element is electrically coupled to a second element there may be further conductive elements between them, and if a first element is electrically connected to a second element there are no conductive elements between them.

The fluid may be susceptible to microbiological growth. The fluid may contain water. The fluid may be a liquid, such as raw milk or raw juice. It is understood that the fluid may contain solid particles. The fluid may be a paste, or a minced or pureed product.

The power source may have a first current-supply terminal. It is understood that the first current-supply terminal can supply a current, such as the current described above, or the first current described below. The power source may also have a second current-supply terminal. It is understood that the first current-supply terminal can supply another current, such as the second current described below.

The power source may have a power-source ground connection that electrically couples the power source to ground. It is understood that that the power-source ground connection forms part of the electric circuit.

It is understood that the power source may be a combined power source. For example, it may comprise a first power source unit and a separate second power source unit, wherein the first power source unit has the first current-supply terminal, and the second power source unit has the second current-supply terminal. It is also understood that the power-source ground connection may be a combined ground connection. For example, it may comprise a first power-source ground connection that electrically couples the first power source unit to ground and a second power-source ground connection that electrically couples the second power source unit to ground.

The housing may be of metal, such as steel. The housing may form, or enclose, an inner space for holding, or retaining, the fluid. The housing may have a wall forming the inner space for holding the fluid. It is understood that the wall is exposed to the fluid. The wall may be electrically conductive. For example, the wall may be of metal. It is understood that the housing, or wall, may be composed of several components that are connected, or joined, together. The wall may be, or form part of the abovementioned portion of the housing. The wall may form part of the electric circuit. This means that at least a portion of the current is conducted through the wall.

The housing may be a static housing, or stationary housing. It is understood that a static housing does not move in use. Alternatively, the housing may have a rotating housing part, or rotary housing part, wherein the rotating housing part is arranged to rotate relative to the surroundings of the housing. It is understood that the rotating housing part forms, or encloses, a part of the inner space. It is further understood that the rotating housing part is exposed to the fluid. It may also form part of the electric circuit, which means that the current is at least partly led to or from ground via the rotating housing part. For example, the housing may comprise an internal connector, such as a bearing or a seal, that electrically connect the rotating housing part to the rest of the housing.

The system may comprise a motor operationally connected to the rotating housing part, for example by a drive shaft of the motor. It is understood that the motor is arranged to rotate the rotating housing part. The motor may be electrically conductive and may form part of the housing-ground connection and/or the housing-support structure described below. More generally, the motor may form part of the electric circuit.

The pipe arrangement may comprise a first pipe, wherein the first pipe is in fluid communication with the housing and arranged to lead the fluid to or from the housing. The first pipe is electrically conductive. For example, it may be of metal, such as steel. The first pipe may be fixed to, or connected directly to, the housing. This means that there is a physical contact between the components. For example, the housing may have, or form, an inlet, or feed nozzle, and the first pipe may be a feed pipe connected directly to the inlet. Here, the inlet is understood to be arranged to receive the fluid from the first pipe and to introduce the fluid into the housing, or into the inner space. It is understood that the first pipe is in fluid communication with the inner space formed by the wall of the housing. The first pipe may form part of the electric circuit. This contributes to an easier installation and to inhibit microbiological growth in at least a portion of the first pipe.

It is understood that the first pipe may be a combined structure. For example, it may be composed of several pipe section that are connected to form the first pipe.

The pipe arrangement may comprise a second pipe, wherein the second pipe is in fluid communication with the housing and arranged to lead the fluid to or from the housing. The second pipe is electrically conductive. For example, it may be of metal, such as steel. The second pipe may be fixed to, or connected directly to, the housing. This means that there is a physical contact between the components. For example, the housing may have, or form, an outlet, or discharge nozzle, and the second pipe may be a discharge pipe connected directly to the outlet. Here, the outlet is understood to be arranged to discharge the fluid from the housing, or from the inner space, and to introduce the fluid into the second pipe. It is understood that the second pipe is in fluid communication with the inner space formed by the wall of the housing. The second pipe may form part of the electric circuit. This contributes to an easier installation and to inhibit microbiological growth in at least a portion of the second pipe.

It is understood that the second pipe may be a combined structure. For example, it may be composed of several pipe section that are connected to form the second pipe.

The first pipe and the second pipe may be spaced apart at the housing. Similarly, the inlet and the outlet of the housing may be spaced apart. The first pipe and the second pipe may connect to the housing at opposite sides of the housing. Similarly, the inlet and the outlet of the housing may be located at opposite sides of the housing. This contributes to a more even distribution of the current in the housing, and in extension a more even reduction of microbiological growth, if the current flows between the first pipe and the second pipe.

It is specified above that the housing may have an inlet and an outlet. It is understood that the housing may have additional inlets and/or additional outlets.

The fluid-processing equipment may comprise: a moving element, or dynamic element, wherein the moving element is located at least partly within the housing, or in the inner space, and the moving element is arranged to move relative to the housing. The moving element may be a rotating element, or rotary element, arranged to rotate relative to the housing. It is understood that the moving element may be a rigid self-supporting structure. It is further understood that the moving element is arranged to be in contact with the fluid.

The moving element may be electrically conductive. For example, it may be of metal, such as steel. The moving element may be electrically coupled, or electrically connected, to the housing. The fluid-processing equipment may comprise an internal connector arranged to electrically couple, or electrically connect, to the moving element to the housing. For example, the internal connector may be a bearing such as a rolling-element bearing, such as an end face mechanical seal. It is understood that the internal connector is electrically conductive. For example, it may be of metal.

The moving element, and if present the internal connector, may form part of the electric circuit. This means that at least a part, or component, of the current is to lead to or from ground via the moving element and the internal connector. This contributes to inhibit microbiological growth also on the moving element.

The system may comprise a motor operationally connected to the moving element, for example by a drive shaft of the motor. It is understood that the motor is arranged to move the moving element. The motor may be electrically conductive and may form part of the housing-ground connection and/or the housing-support structure described below. More generally, the motor may form part of the electric circuit.

The system may comprise a housing-support structure that contacts and supports the housing. It is specified above that the housing may be a static housing, and the housing-support structure may be fixed to the housing. It is also specified above that the housing may have a rotating housing part and the housing-support structure may rotationally support the rotating housing part, for example by way of one or more bearings forming part of the housing-support structure.

The system may comprise a first pipe-support structure that contacts and supports the first pipe. The first pipe support-structure may be fixed to the first pipe. For example, it may comprise a clamp attached to the first pipe. It is understood that the first pipe-support structure may be spaced apart from the housing-support structure at the first pipe.

Additionally, the system may comprise a second pipe-support structure that contacts and supports the second pipe. The second pipe-support structure may be fixed to the second pipe. For example, it may comprise a clamp attached to the second pipe. It is understood that the second pipe-support structure may be spaced apart from the housing-support structure and/or the first pipe support structure at the second pipe.

It is further understood that the support structures may be connected directly to the housing, first pipe, respectively second pipe. This means that there are no other components between the housing-support structure and the housing, between the first pipe-support structure and the first pipe, and between the second pipe-support structure and the second pipe.

Any of the housing-support structure, the first pipe support structure, and the second pipe support structure may be electrically conductive. For example, the support structure may be of metal, such as steel.

Any of the housing-support structure, the first pipe-support structure, and the second pipe-support structure may electrically insulate the housing, first pipe, respectively the second pipe from ground. This means that a current cannot go to ground via the support structure. The support structure may have an insulating spacer, or insulating element, that insulates the housing, first pipe, or second pipe from ground. For example, the support structure may comprise a clamp, wherein the clamp holds the housing, first pipe, or second pipe and the insulating spacer is positioned between the clamp and the supported element.

It is understood that the above support structures support the housing, first pipe, respective second pipe relative to the surroundings. The support structures may form part of a combined support structure.

The housing as such may be electrically insulated from ground. For example, the first pipe and the second pipe may be arranged to carry the full weight of the fluid-processing equipment, or the full weight of the housing. Worded differently, the housing may be supported by the first pipe and the second pipe alone. It is specified above that the system may comprise a housing-support structure. The housing-support structure may then electrically insulate the housing from ground.

The system may comprise a housing-ground connection that is connected to the housing and electrically connects the housing to ground. It is understood that the housing-ground connection can lead a current between the housing and ground. For example, the housing-support structure may form, or form part of, the housing-ground connection. Additionally or alternatively, the system may comprise an electric wire coupled, or connected, to the housing and to the ground that form, or form part of, the housing-ground connection. The housing-ground connection may form part of the electric circuit, as is further discussed below.

The system may comprise a first pipe-ground connection that is connected to the first pipe and electrically connects the first pipe to ground. It is understood that first pipe-ground connection can lead a current between the first pipe and ground. For example, the first pipe-support structure may form, or form part of, the first pipe-ground connection. Additionally or alternatively, the system may comprise an electric wire coupled, or connected, to the first pipe and to the ground that form, or form part of, the first pipe-ground connection. The first pipe-ground connection may form part of the electric circuit, as is further discussed below.

The system may comprise a second pipe-ground connection connected to the second pipe and that electrically connects the second pipe to ground. It is understood that second pipe-ground connection can lead a current between the second pipe and ground. For example, the second pipe-support structure may form, or form part of, the second pipe-ground connection. Additionally or alternatively, the system may comprise an electric wire coupled, or connected, to the second pipe and to the ground that form, or form part of, the second pipe-ground connection. The second pipe-ground connection may form part of the electric circuit, as is further discussed below.

It is understood that a ground connection may comprise several independent connections to ground. For example, it may comprise two separate electric wires or support structures connecting the housing to ground.

It is specified above that the pipe arrangement may comprise a first pipe. The first pipe may form part of the electric circuit. The power source may be electrically coupled, or electrically connected, to the first pipe, the first pipe may be electrically coupled, or electrically connected, to the housing, and the housing may be electrically coupled, or electrically connected, to ground. It is understood that electric circuit is thus arranged to lead the current through the first pipe and the housing.

The current may be supplied by, or via, the first current-supply terminal of the power source.

It is specified above that the pipe arrangement may comprise a first pipe and a housing-ground connection. The first pipe and the housing-ground connection may form part of the electric circuit. The power source may be electrically coupled, or electrically connected, to the first pipe, the first pipe may be electrically coupled, or electrically connected, to the housing, and the housing may be electrically coupled, or electrically connected, to the housing-ground connection. It is understood that the housing-ground connection is electrically coupled, or electrically connected, to ground. It is further understood that electric circuit is thus arranged to lead the current through the first pipe and the housing.

The electric circuit may be arranged to lead the current through at least a portion of the first pipe. This means that there may be parts of the first pipe through which the current does not run. The electric circuit may be arranged to lead the current through the complete first pipe.

The current may be supplied by, or via, the first current-supply terminal of the power source.

It is specified above that the pipe arrangement may have a first pipe and a second pipe. The first pipe and the second pipe may form part of the electric circuit. It is further specified that the housing as such may be electrically insulated from ground. The power source may be electrically coupled, or electrically connected, to the first pipe, the first pipe may be electrically coupled, or electrically connected, to the housing, the housing may be electrically coupled, or electrically connected, to the second pipe, and the second pipe may be electrically coupled, or electrically connected, to ground. It is understood that electric circuit is thus arranged to lead the current through the first pipe, the housing, and the second pipe.

The current may be supplied by, or via, the first current-supply terminal of the power source.

It is specified above that the pipe arrangement may have a first pipe, a second pipe, and a housing-ground connection. The first pipe, the second pipe, and the housing-ground connection may form part of the electric circuit. The power source may be electrically coupled, or electrically connected, to the first pipe, the first pipe may be electrically coupled, or electrically connected, to the housing, the housing may be electrically coupled, or electrically connected, to the second pipe and to the housing-ground connection, and the second pipe may be electrically coupled, or electrically connected, to ground. It is understood that the housing-ground connection is electrically coupled, or electrically connected, to ground. It further is understood that the electric circuit is thus arranged to lead the current through the first pipe and the housing, to lead an electric first current component through the housing-ground connection, and to lead an electric second current component through the second pipe, wherein the combined first current component and the second current component correspond to, or constitute, the current.

The current may be supplied by, or via, the first current-supply terminal of the power source.

It is specified above that the pipe arrangement may have a first pipe, a second pipe, and a housing-ground connection. The first pipe, the second pipe, and the housing-ground connection may form part of the electric circuit. The power source may be electrically coupled, or electrically connected, to the first pipe and to the second pipe, the first pipe may be electrically coupled, or electrically connected, to the housing, the second pipe may be electrically coupled, or electrically connected, to the housing, the housing may be electrically coupled, or electrically connected, to the housing-ground connection. It is understood that the housing-ground connection is electrically coupled, or electrically connected, to ground.

It is specified above that the power source is arranged to supply an electric current, and that the housing, pipe arrangement, and the power source are operationally coupled in an electric circuit connected to ground and arranged to lead the current to or from ground via the pipe arrangement and the housing. Worded differently, the power source may be arranged to supply an electric first current and an electric second current, and the housing, pipe arrangement, and the power source may be operationally coupled in an electric circuit connected to ground and arranged to lead the first current and the second current to or from ground via the pipe arrangement and the housing.

It is understood that the electric circuit is thus arranged to lead the first current through the first pipe, the housing and the ground connection, and to lead the second current through the second pipe, the housing, and the ground connection. It is further understood that the first current and the second current are combined at, or in, the housing, to a combined current that is conducted to ground via the housing-ground connection.

The first current and the second current may be supplied by, or via, the first current-supply terminal of the power source. Alternatively, the first current may be supplied by, or via, the first current-supply terminal of the power source, and the second current may be supplied by, or via, the second current-supply terminal of the power source.

The system may further comprise: a first connector, or first connector arrangement, connected to the first pipe, wherein the first pipe is electrically coupled to the power source via the first connector. This means that the first connector forms part of the abovementioned electric circuit. The electric circuit may be arranged to lead the current, or first current, via the first connector.

It is specified above that the system may comprise a first pipe-ground connection that is connected to the first pipe and electrically connects the first pipe to ground. The first connector and the first pipe-ground connection may be spaced apart. The first connector may be connected to the first pipe between the first pipe-ground connection and the housing.

The first connector may be located closer in the electric circuit to the housing, housing-ground connection, second pipe, and/or second pipe-ground connection than to the first pipe-ground connection. Worded differently, the internal path length within the first pipe between the first connector and the housing may be shorter than the internal path length within the first pipe between the first connector and the first pipe-ground connection. Similarly, the internal path length within the first pipe and the housing between the first connector and the housing-ground connection may be shorter than the internal path length within the first pipe between the first connector and the first pipe-ground connection. Similarly, the internal path length within the first pipe and the housing between the first connector and the second pipe may be shorter than the internal path length within the first pipe between the first connector and the first pipe-ground connection. Similarly, the internal path length within the first pipe, housing, and second pipe between the first connector and the second pipe-ground connection may be shorter than the internal path length within the first pipe between the first connector and the first pipe-ground connection. The features presented here contribute to reduce currents not leading through the housing, which contributes to an improved efficiency.

It is understood that the power source may be arranged to supply the current and an additional current, or the first current and an additional first current, and that the electric circuit may further be arranged to lead the additional current, or additional first current, to or from ground via the first connector, the first pipe, and the first pipe-ground connection. Effectively, this means the electric circuit is arranged to lead the combined current and additional current, or the combined first current and additional first current, via the first connector, and the current and the additional current, or the first current and the additional first current, are divided at the first connecter, and the electric circuit is arranged to lead the current, or the first current, to or from ground via the housing and to lead the additional current, or the additional first current, to or from ground via the first pipe-ground connection.

The system may further comprise: a second connector, or second connector arrangement connected to the second pipe, wherein the second pipe is electrically coupled to the power source via the second connector. This means that the second connector forms part of the abovementioned electric circuit. The electric circuit may be arranged to lead the second current via the second connector.

It is specified above that the system may comprise a second pipe-ground connection that is connected to the second pipe and electrically connects the second pipe to ground. The second connector may be connected to the second pipe between the second pipe-ground connection and the housing. The second connector and the second pipe-ground connection may be spaced apart.

The second connector may be located closer in the electric circuit to the housing, or the housing-ground connection than to the second pipe-ground connection. Worded differently, the internal path length within the second pipe between the second connector and the housing may be shorter than the internal path length within the second pipe between the second connector and the second pipe-ground connection. Similarly, the internal path length within the second pipe and the housing between the second connector and the housing-ground connection may be shorter than the internal path length within the second pipe between the second connector and the second pipe-ground connection. The features presented here contribute to reduce currents not leading through the housing, which contributes to an improved efficiency.

It is understood that the power source may be arranged to supply the second current and an additional second current, and that the electric circuit may further be arranged to lead the additional second current to or from ground via the second connector, the second pipe, and the second pipe-ground connection. Effectively, this means the electric circuit is arranged to lead the combined second current and additional second current, via the second connector, and the second current and the additional second current are divided at the second connecter, and the electric circuit is arranged to lead the second current to or from ground via the housing and to lead the additional second current to or from ground via the second pipe-ground connection.

The system may further comprise an electric first wire, wherein the first wire connects the first connector to the power source. It is understood that the current, the current and the additional current, the first current, or the first current and additional first current are conducted via the first wire. The first wire may be connected to the abovementioned first current-supply terminal of the power source. The system may further comprise an electric second wire, wherein the second wire connects the second connector to the power source. It is understood that the second current or the second current and additional second current are conducted via the second wire. The second wire may be connected to the abovementioned first current-supply terminal or to the second current-supply terminal of the power source.

Any of the current, first current, and second current may be an alternating current. An alternating current is understood to periodically change direction. The alternating current have a square waveform. The alternating current may be below 10 mA, below 1 mA, or below 0.1 mA. Any of the currents may be greater than 0.01 mA. The alternating current may be in the range 10 mA to 1 mA, 1 mA to 0.1 mA, or 0.1 mA to 0.01 mA. Here, the alternating current is understood as the absolute maximum over a period. It has been found that this current is sufficient for inhibiting microbiological growth. The alternating current may have a frequency below 100 Hz, below 10 Hz, or below 1 Hz. The frequency may be greater than 0.1 Hz. The alternating current may be supplied at a peak voltage below 120 V, in the range 10 V to 100 V, or in the range 20 V to 90 V, or in the range 30 V to 70 V.

If the current, first current, and second current are alternating currents, the additional current, additional first current, and additional second current may also be alternating currents.

Alternatively to the current, first current, and second current being an alternating current, any of the currents may be a direct current. A direct current is understood to have a constant direction. The direct current may be a pulsed current. It is understood that no current is supplied between pulses. The pulsed current may have a regular pulse frequency. The pulse frequency may be below 100 Hz, below 10 Hz, or below 1 Hz. The pulse frequency may be greater than 0.1 Hz. The direct current may have a duty cycle in the range 60% to 40%, or about 50%. For example, the current may be supplied with a pulse length of 500 ms with a pulse separation of 500 ms. The pulsed direct current may be supplied at a peak voltage below 120 V, in the range 10 V to 100 V, or in the range 20 V to 90 V, or in the range 30 V to 70 V.

If the current, first current, and second current are direct currents, the additional current, additional first current, and additional second current may also be direct currents.

It is specified that the fluid-processing equipment has a housing arranged to hold a fluid. Examples of such equipment are presented below. It is understood that the fluid-processing equipment may comprise several functionally different or independent equipment units, and that the equipment units are fluidly connected and jointly form the housing. For example, the fluid-processing equipment may be composed of a centrifugal pump, a pipe, and a centrifuge separator. The pipe interconnects the pump and the separator. The pump forms the inlet described above, the separator forms the outlet described above, and the pump, pipe, and separator jointly form the housing.

The fluid-processing equipment may be, or comprise, a pump forming the housing, or at least a part of the housing. It is understood that the pump has a moveable element, for example as described above. For example, the pump may be a centrifugal pump having volute casing forming at least a part of the housing. The moving element is a rotating element and comprises an impeller located within the casing and arranged to cooperate with the casing to pump the fluid. It is understood that the volute casing forms a volute space that forms part of the inner space of the housing, and that impeller is located in the volute space. In this example, it is understood that the housing is a static housing.

The fluid-processing equipment may be, or comprise, a centrifuge separator forming the housing. The housing may have a rotating housing part, as specified above. For example, the centrifuge separator may have a rotary casing, or bowl, forming part the rotating housing part.

### Brief description of the drawings

A more complete understanding of the abovementioned and other features and advantages of the technology proposed herein will be apparent from the following detailed description of preferred embodiments in conjunction with the appended drawings, wherein:
Fig. 1 is a schematic view of a system with a fluid-processing equipment, a pipe arrangement, and an electrical power source,
Fig. 2 is a schematic view of another system with a fluid-processing equipment, a pipe arrangement, and an electrical power source,
Fig. 3 is a schematic view of another system with a fluid-processing equipment, a pipe arrangement, and an electrical power source,
Fig. 4 is a schematic view of another system with a fluid-processing equipment, a pipe arrangement, and an electrical power source, and
Fig. 5 is a schematic view of another system with a fluid-processing equipment, a pipe arrangement, and an electrical power source,
Fig. 6 is a schematic cross-sectional view of a processing equipment, and
Fig. 7 is a schematic cross-sectional view of another processing equipment.

### Detailed description

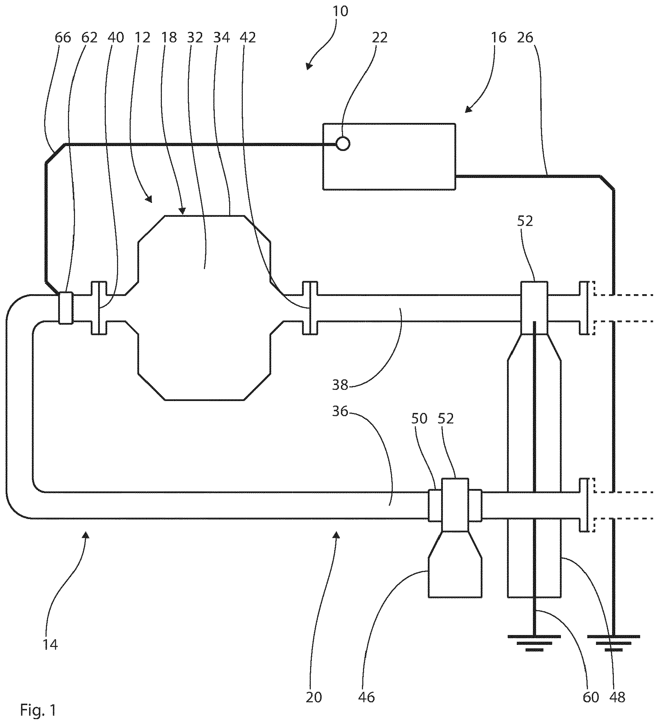

A system 10 is shown in Fig. 1 having a fluid-processing equipment 12, a pipe arrangement 14, and an electrical power source 16. The fluid-processing equipment 12 has an electrically conductive housing 18 of steel that can hold a fluid. The housing 18 has a wall 34 exposed to the fluid and forming an inner space 32 for holding the fluid.

The pipe arrangement 14 has an electrically conductive first pipe 36 of steel that is connected directly to an inlet 40 formed by the housing 18. The first pipe 36 is in fluid communication with the housing 18 and arranged to lead the fluid to the housing 18. The inlet 40 is arranged to receive the fluid from the first pipe 36 and to introduce the fluid into the inner space 32 of the housing 18. Similarly, the pipe arrangement 14 has an electrically conductive second pipe 38 of steel that is connected directly to an outlet 42 formed by the housing 18. The inlet 40 and the outlet 42 of the housing 18 are be spaced apart, which means that the first pipe 36 and the second pipe 38 are spaced apart at the housing 18. The second pipe 38 is in fluid communication with the housing 18 and arranged to lead the fluid from the housing 18. The outlet 42 is arranged discharge the fluid from the inner space 32 of the housing 18 and to introduce the fluid into the second pipe 38. This way, the pipe arrangement 14 is electrically conductive and electrically coupled to the housing 18, the pipe arrangement 14 is arranged to lead the fluid to and from the housing 18, and the fluid-processing equipment 12 and the pipe arrangement 14 are arranged to allow a flow of the fluid through the housing 18.

The system 10 has a first pipe-support structure 46 that directly contacts and supports the first pipe 36 and a second pipe-support structure 48 that directly contacts and supports the second pipe 38. The support structures and are spaced apart at the housing 18. The first pipe-support structure 46 and the second pipe-support structure 48 are respectively fixed to the first pipe 36 and the second pipe 38 by a clamp 52. The first pipe 36 and the second pipe 38, and in extension the first pipe-support structure 46 and the second pipe-support structure 48, carry the full weight of the fluid-processing equipment 12 that includes the housing 18. This way, the housing 18 as such is electrically insulated from ground. In an alternative embodiment, the system 10 has a housing-support structure 44 that supports the housing 18 and electrically insulates the housing 18 from ground.

The first pipe-support structure 46 has an insulating spacer 50 between the clamp 52 and the first pipe 36 that insulates the first pipe 36 from ground. The second pipe-support structure 48 is electrically conductive and forms a second pipe-ground connection 60 that connects the second pipe 38 to ground. In an alternative embodiment, the second pipe-support structure 48 has an insulating element similar to the first pipe-support structure 46, and an electric wire connected to the second pipe 38 forms the second pipe-ground connection 60.

The power source 16 has a first current-supply terminal 22 by which it can supply an electric current. The power source 16 further has a power-source ground connection 26 that electrically couples the power source 16 to ground. The system 10 has a first connector 62 in the form of a clamp connected to the first pipe 36 and an electric first wire 66 that connects the first connector 62 to the first current-supply terminal 22. This way, the first pipe 36 is electrically coupled to the power source 16 via the first connector 62.

The power-source ground connection 26, first wire 66, first connector 62, first pipe 36, housing 18, second pipe 38, and second pipe-support structure 48 form an electric circuit 20. Effectively, the second pipe 38 and the second pipe-support structure 48 constitutes a housing-ground connection 56, and the housing 18, pipe arrangement 14, and the power source 16 are operationally coupled in an electric circuit 20 connected to ground and arranged to lead the current to or from ground via the pipe arrangement 14 and the housing 18.

The first current-supply terminal 22 of the power source 16 can supply an alternating current with a square waveform. The current is about 0.5 mA, has a frequency below of 1 Hz, and is supplied at a peak voltage of about 50 V. The supplied current contributes to reduce microbiological growth in the housing 18. It also contributes to reduce microbiological growth in the first pipe 36 between the first connector 62 and the housing 18 and in the second pipe 38 between the housing 18 and the second pipe-support structure 48 forming the second pipe-ground connection 60.

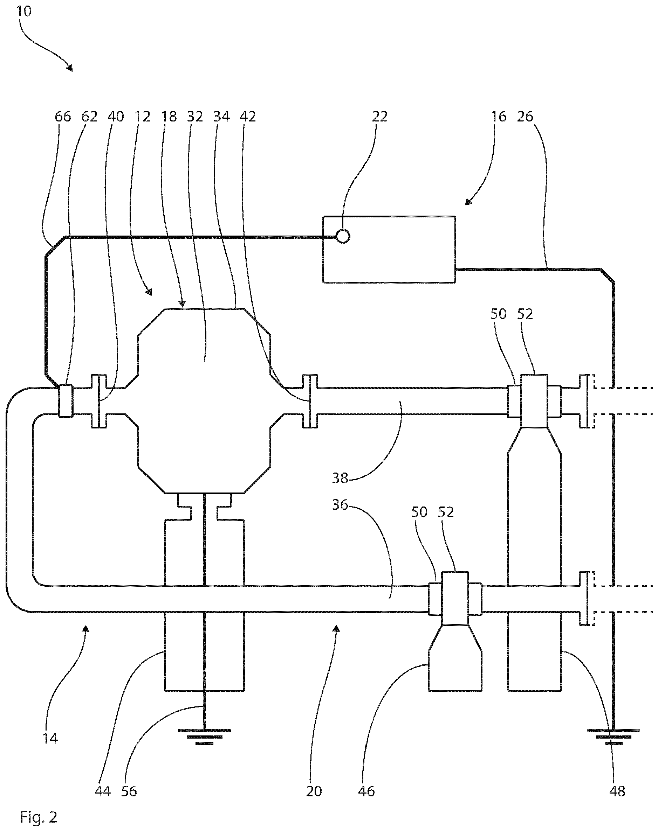

Another system 10 is shown in **Fig. 2**, it differs from the system 10 of Fig. 1 by having a housing-support structure 44 that directly contacts and supports the housing 18. The support structures 44, 46, and 48 are spaced apart at the housing 18. The housing-support structure 44 is attached to the housing 18 by a screw (not shown). The housing-support structure 44 is electrically conductive and forms a housing-ground connection 56 that connects the housing 18 to ground. In an alternative embodiment, the system 10 has no housing-support structure 44, as in the embodiment of Fig. 1. Instead, an electric wire connected to the housing 18 forms the housing-ground connection 56.

The embodiment further differs by also the second pipe-support structure 48 having an insulating spacer 50 between the clamp 52 and the second pipe 38 that insulates the second pipe 38 from ground.

The power-source ground connection 26, first wire 66, first connector 62, first pipe 36, housing 18, and housing-support structure 44 form an electric circuit 20. Effectively, the housing 18, pipe arrangement 14, and the power source 16 are operationally coupled in an electric circuit 20 connected to ground and arranged to lead the current to or from ground via the pipe arrangement 14 and the housing 18.

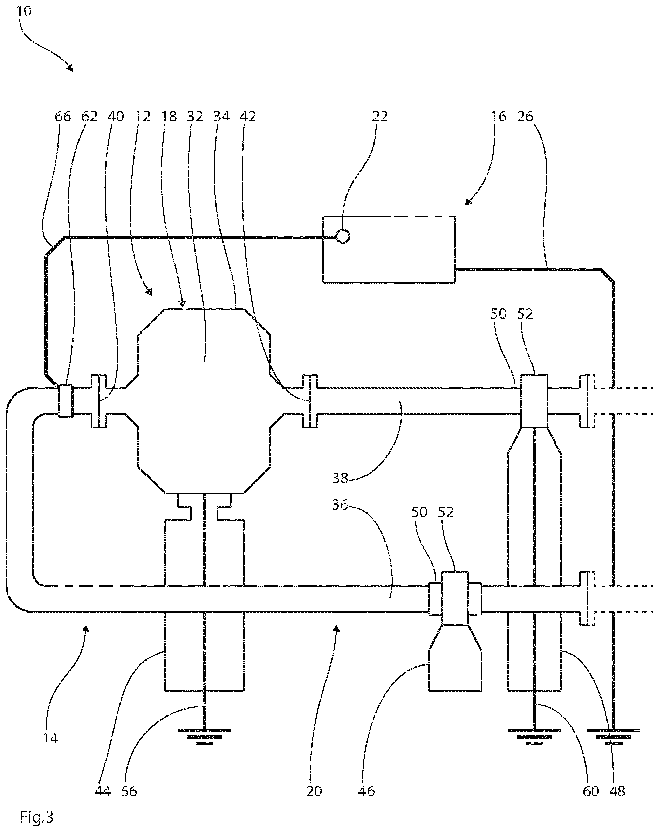

Another system 10 is shown in **Fig. 3**, it differs from the system 10 of Fig. 1 by having a housing-support structure 44 that directly contacts and supports the housing 18. The support structures 44, 46, and 48 are spaced apart at the housing 18. The housing-support structure 44 is attached to the housing 18 by a screw (not shown). The complete housing-support structure 44 is electrically conductive and forms a housing-ground connection 56 that connects the housing 18 to ground. In an alternative embodiment, the system 10 has no housing-support structure 44, as in the embodiment of Fig. 1. Instead, an electric wire connected to the housing 18 forms the housing-ground connection 56.

The power-source ground connection 26, first wire 66, first connector 62, first pipe 36, housing 18, and housing-support structure 44, second pipe 38, and second pipe-support structure 48 form an electric circuit 20 with the housing-support structure 44 coupled in parallel with the second pipe 38 and the second pipe-support structure 48. Effectively, the housing 18, pipe arrangement 14, and the power source 16 are operationally coupled in an electric circuit 20 connected to ground and arranged to lead the current to or from ground via the pipe arrangement 14 and the housing 18. The current is split into a first current component and a second current component at the housing 18, with the first current component being led through the housing-ground connection 56 and the second current component being led through the second pipe 38 and the second pipe-support structure 48.

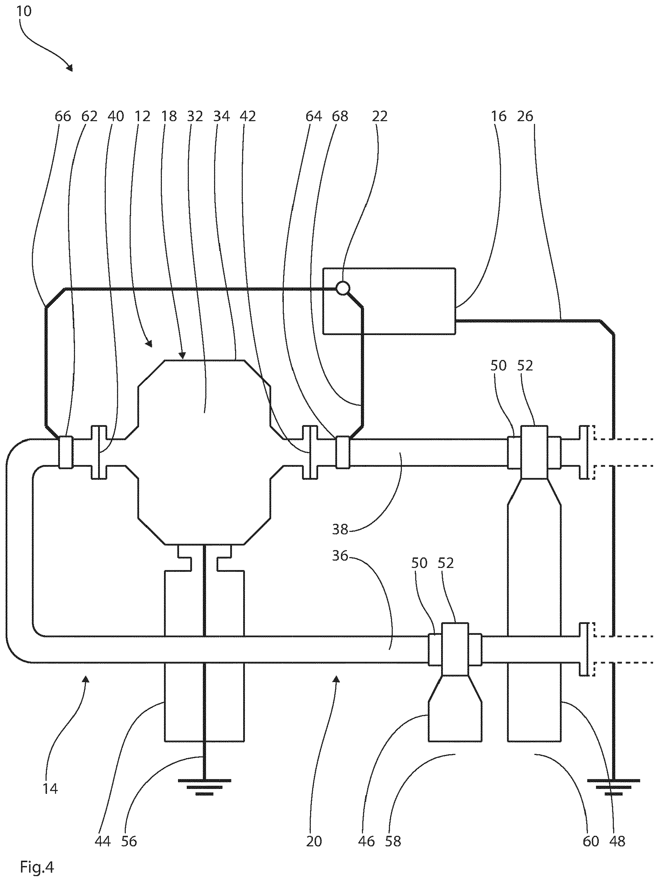

Another system 10 is shown in **Fig. 4**, it differs from the system 10 of Fig. 2 by further having a second connector 64 in the form of a clamp connected to the second pipe 38 and an electric second wire 68 that connects the second connector 64 to the first current-supply terminal 22 of the power source 16. The power source 16 can supply a first current via the first connector 62 and a second current via the second connector 64. In an alternative embodiment, the electric second wire 68 is connected to second current-supply terminal of the power supply that supplies the second current.

The power-source ground connection 26, first wire 66, first connector 62, first pipe 36, second wire 68, second connector 64, second pipe 38, housing 18, and housing-support structure 44 form an electric circuit 20 with the first wire 66, first connector 62, and first pipe 36 coupled in parallel with the second wire 68, second connector 64, and second pipe 38. This means that both the first current and the second current pass through the housing 18 and are combined in the housing 18 to a current that is led to ground via the housing-ground connection 56. Effectively, the power source 16 is operationally coupled in an electric circuit 20 connected to ground and arranged to lead the first current and the second current to or from ground via the pipe arrangement 14 and the housing 18.

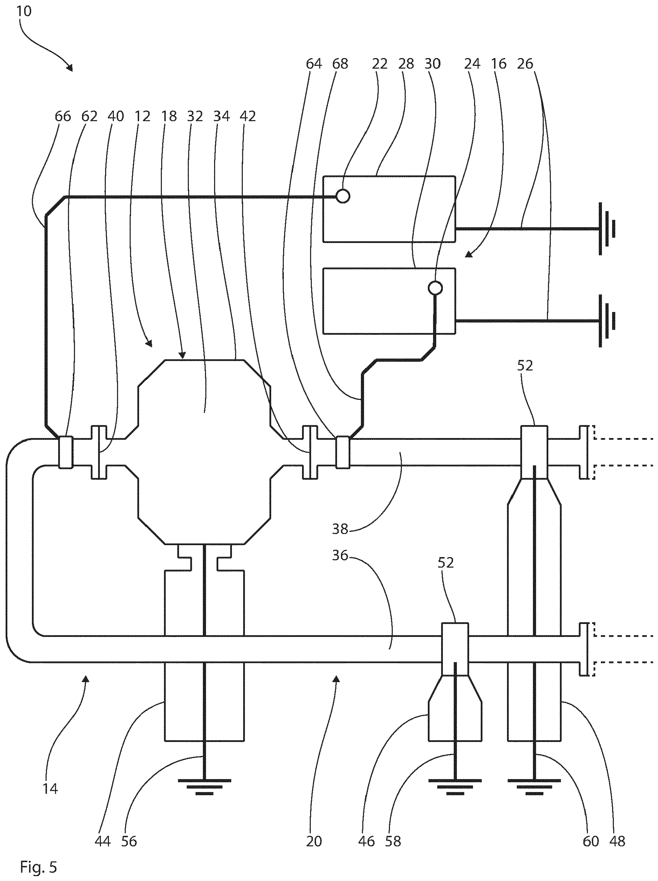

Another system 10 is shown in **Fig. 5**, it differs from the system 10 of Fig. 4 by the first pipe-support structure 46 and the second pipe-support structure 48 having no insulating elements and respectively forming a first pipe-ground connection 58 and a second pipe-ground connection 60. The first pipe-ground connection 58 couples the first pipe 36 to ground and the second pipe-ground connection 60 couples the second pipe 38 to ground.

The embodiment further differs by the power supply having a first power source unit 28 and a second power source unit 30 that are both connected to ground by the power-source ground connection 26. The first power source unit 28 has the first current-supply terminal 22 and the second power source unit 30 has a second current-supply terminal 24. The first wire 66 is connected to the first current-supply terminal 22 and the second wire 68 is connected to the second current-supply terminal 24. The first current is supplied by the first current-supply terminal 22 and the second current is supplied by the second current-supply terminal 24. The first current-supply terminal 22 also supplies an additional first current and the second current-supply terminal 24 also supplies an additional second current.

This way, the electric circuit 20 is arranged to lead the first current to or from ground via the first connector 62, the first pipe 36, the housing 18, and the housing-ground connection 56, and to lead the second current to or from ground via the second connector 64, the second pipe 38, the housing 18, and the housing-ground connection 56, as in the embodiment of Fig. 4. The electric circuit 20 is further arranged to lead the additional first current to or from ground via the first connector 62, the first pipe 36, and the first pipe-ground connection 58, and to lead the additional second current to or from ground via the second connector 64, the second pipe 38, and the second pipe-ground connection 60.

The first connector 62 and the first pipe-ground connection 58 are space apart. The first connector 62 is connected to the first pipe 36 between the first pipe-ground connection 58 and the housing 18. The internal path length within the first pipe 36 and the housing 18 between the first connector 62 and the housing-ground connection 56 is shorter than the internal path length within the first pipe 36 between the first connector 62 and the first pipe-ground connection 58. The internal path length within the first pipe 36, the housing 18, and the second pipe 38 between the first connector 62 and the second pipe-ground connection 60 is shorter than the internal path length within the first pipe 36 between the first connector 62 and the first pipe-ground connection 58.

The second connector 64 and the second pipe-ground connection 60 are space apart. The second connector 64 is connected to the second pipe 38 between the second pipe-ground connection 60 and the housing 18. The internal path length within the second pipe 38 and the housing 18 between the second connector 64 and the housing-ground connection 56 is shorter than the internal path length within the second pipe 38 between the second connector 64 and the second pipe-ground connection 60.

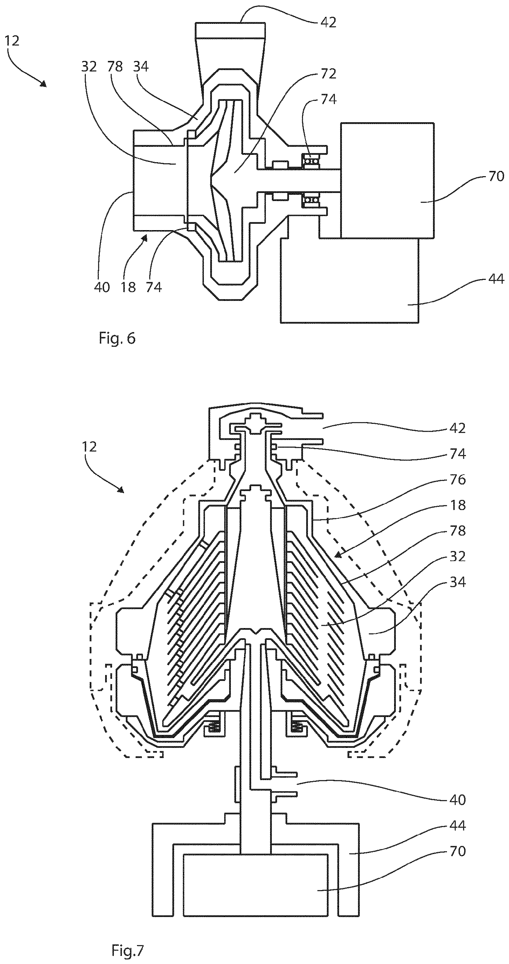

A fluid-processing equipment 12 in the form of a centrifugal pump that can pump a liquid is shown in **Fig. 6****.** The centrifugal pump can be used in any of the systems described in relation to Figs. 1 to 5. The fluid-processing equipment 12 can change the dynamic properties of the liquid. More precisely, it can increase the pressure and the flow rate of the liquid.

The fluid-processing equipment 12 has a static housing 18 and the housing-support structure 44 is fixed to the housing 18. The housing 18 has a wall 34 exposed to the liquid and enclosing an inner space 32 for holding the liquid. It forms an inlet 40 that can be connected to the first pipe and an outlet 42 that can be connected to the second pipe. The inlet 40 and the outlet 42 of the housing 18 are be spaced apart. The inlet 40 introduces the liquid into the inner space 32 and the outlet 42 discharges the liquid from the inner space 32. This way, the fluid-processing equipment 12 is arranged to allow a flow of the liquid through the housing 18. The housing 18 is of steel and is electrically conductive.

The wall 34 of the housing 18 has a portion 78 that is exposed to the liquid and a current passing between the inlet 40 and the outlet 42 will pass through the portion 78. This means that the portion 78 forms part of the abovementioned electric circuit 20.

The fluid-processing equipment 12 has an electrically conductive moving element 72, or more precisely a rotating element, of steel. The moving element 72 is a shaft and an impeller that is partly located within the inner space 32 of the housing 18. The moving element 72 is rotated relative to the static housing 18 by a motor 70. The moving element 72 is electrically conductive and electrically coupled to the housing 18 by an internal connector 74 in the form of a wear ring and a rolling-element bearing. This way, a part of the current passes through the moving element 72 and it forms part of the electric circuit 20.

Another fluid-processing equipment 12 in the form of a centrifuge separator is shown in **Fig. 7****.** The centrifuge separator can be used in any of the systems described in relation to Figs. 1 to 5, but with the first pipe 36 connected to the outlet 42 and the second pipe 38 connected to the inlet 40. The fluid is a liquid and the fluid-processing equipment 12 can change inherent properties of the liquid. More precisely, it can form a lighter phase and a heavier phase of the liquid.

The housing 18 has a rotating housing part 76, or bowl, that can rotate relative to the surroundings of the housing 18. The rotating housing part 76 encloses a part of the inner space 32 and is exposed to the liquid. The housing 18 has internal connectors 74 in the form of bearings or seals that connect the rotating housing part 76 to the rest of the housing 18. This way, the rotating housing part forms part of the electric circuit 20 and the current is at least partly led to or from ground via the rotating housing part 76.

The housing 18 has a wall 34 exposed to the liquid and enclosing an inner space 32 for holding the liquid. The inlet 40 and the outlet 42 of the housing 18 are be spaced apart. The inlet 40 introduces the liquid into the inner space 32 and the outlet 42 discharges the liquid from the inner space 32. This way, the fluid-processing equipment 12 is arranged to allow a flow of the liquid through the housing 18. The housing 18 is of steel and is electrically conductive.

The rotating housing part 76 is rotated by a motor 70 forming part of the housing-support structure 44. In the embodiments of Figs. 2 to 5 the motor 70 is electrically conductive, forms part of the housing-support structure 44 and the electric circuit 20, and the current is conducted to ground via the motor 70.

In an alternative embodiment, the fluid-processing equipment is composed a centrifugal pump, a pipe, and a centrifuge separator fluidly coupled in series and jointly forming the housing arranged to hold a fluid with the pipe interconnecting the pump and the separator. The pump forms the inlet of the housing, and the separator forms the outlet of the housing.

### Item list

10 system
12 fluid-processing equipment
14 pipe arrangement
16 electric power source
18 housing
20 electric circuit
22 first current-supply terminal
24 second current-supply terminal
26 power-source ground connection
28 first power source unit
30 second power source unit
32 inner space
34 wall
36 first pipe
38 second pipe
40 inlet
42 outlet
44 housing-support structure
46 first pipe-support structure
48 second pipe-support structure
50 insulating spacer
52 clamp
56 housing-ground connection
58 first pipe-ground connection
60 second pipe-ground connection
62 first connector
64 second connector
66 electric first wire
68 electric second wire
70 motor
72 moving element
74 internal connector
76 rotating housing part
78 exposed portion

## Claims

1. A system (10) that comprises:
- a fluid-processing equipment (12),
- a pipe arrangement (14), and
- an electric power source (16), wherein
the fluid-processing equipment (12) has a housing (18) arranged to hold a fluid, the pipe arrangement (14) is arranged to lead the fluid to and from the housing (18), the housing (18) is electrically conductive, the pipe arrangement (14) is electrically conductive and electrically coupled to the housing (18), the power source (16) is arranged to supply an electric current, the housing (18), pipe arrangement (14), and the power source (16) are operationally coupled in an electric circuit (20) connected to ground and arranged to lead the current to or from ground via the pipe arrangement (14) and the housing (18), and the current is configured to inhibit microbiological growth within the housing (18).

2. The system (10) according to claim 1, wherein the housing (18) is a static housing, and the fluid-processing equipment (12) comprises: a moving element (72), the moving element (72) is located at least partly within the housing (18), and the moving element (72) is arranged to move relative to the housing (18).

3. The system (10) according to claim 1 or 2, wherein the housing (18) has a rotating housing part (76), and the rotating housing part (76) is arranged to rotate relative to the surroundings of the housing (18).

4. The system (10) according to any of the claims 1 to 3, wherein the pipe arrangement (14) comprises an electrically conductive first pipe (36), the first pipe (36) is in fluid communication with the housing (18) and arranged to lead the fluid to or from the housing (18), and the first pipe (36) is electrically conductive and forms part of the electric circuit (20).

5. The system (10) according to claim 4, wherein the pipe arrangement (14) comprises a second pipe (38), the second pipe (38) is in fluid communication with the housing (18) and arranged to lead the fluid to or from the housing (18), and the second pipe (38) is electrically conductive and forms part of the electric circuit (20).

6. The system (10) according to any of the claims 1 to 5, wherein the system (10) further comprises a housing-ground connection (56) that is connected to the housing (18) and electrically connects the housing (18) to ground, and the housing-ground connection (56) forms part of the electric circuit (20).

7. The system (10) according to any of the claim 1 to 6, wherein the system (10) further comprises a first pipe-ground connection (58) that is connected to the first pipe (36) and electrically connects the first pipe (36) to ground, and the first pipe-ground connection (58) forms part of the electric circuit (20).

8. The system (10) according to claim 7, wherein the system (10) further comprises: a first connector (62) connected to the first pipe (36), and the first pipe (36) is electrically coupled to the power source (16) via the first connector (62), the first connector (62) and the first pipe-ground connection (58) are spaced apart, and the first connector (62) is connected to the first pipe (36) between the first pipe-ground connection (58) and the housing (18).

9. The system (10) according to claim 8, wherein the first connector (62) is located closer in the electric circuit (20) to the housing (18) than to the first pipe-ground connection (58).

10. The system (10) according to any of the claim 4, wherein the power source (16) is electrically coupled to the first pipe (36), the first pipe (36) is electrically coupled to the housing (18), and the housing (18) is electrically coupled to ground.

11. The system (10) according to claim 5, wherein the housing (18) as such is electrically insulated from ground, the power source (16) is electrically coupled to the first pipe (36), the first pipe (36) is electrically coupled to the housing (18), the housing (18) is electrically coupled to the second pipe (38), and the second pipe (38) is electrically coupled to ground.

12. The system (10) according to claims 4 and 6, wherein the power source (16) is electrically coupled to the first pipe (36), the first pipe (36) is electrically coupled to the housing (18), and the housing (18) is electrically coupled to the housing-ground connection (56), and the housing-ground connection (56) is electrically coupled to ground.

13. The system (10) according to claims 5 and 6, wherein the power source (16) is electrically coupled to the first pipe (36), the first pipe (36) is electrically coupled to the housing (18), the housing (18) is electrically coupled to the second pipe (38) and to the housing-ground connection (56), and the second pipe (38) and the housing-ground connection (56) is electrically coupled to ground.

14. The system (10) according to claim 5, wherein the power source (16) is electrically coupled to the first pipe (36) and the second pipe (38), the first pipe (36) is electrically coupled to the housing (18), the second pipe (38) is electrically coupled to the housing (18), the housing (18) is electrically coupled to the housing-ground connection (56), and the housing-ground connection (56) is electrically coupled to ground.

15. The system (10) according to any of the claims 1 to 14, wherein the current is an alternating current, the current has a square waveform, the current is below 10 mA, and the current is greater than 0.01 mA.
